# EUROPEAN PATENT SPECIFICATION

(11) **EP 1 984 007 B1**
(45) Date of publication and mention of the grant of the patent: **19.08.2015**
(21) Application number: 07701775.4
(22) Date of filing: 09.02.2007
(51) Int. Cl.: A61K 35/76, A61N 5/00, A61P 35/00

(54) **USE OF LOW DOSE LOCAL IMMUNE SUPPRESSION TO ENHANCE ONCOLYTIC VIRAL THERAPY**
VERWENDUNG VON NIEDRIGDOSIERTEM LOKALER IMMUNSUPPRESSION ZUR VERSTÄRKUNG EINER ONKOLYTISCHEN VIRALEN THERAPIE
UTILISATION D'IMMUNOSUPPRESSION LOCALE À FAIBLE DOSE POUR AMÉLIORER UNE THÉRAPIE VIRALE ONCOLYTIQUE

(30) Priority: 13.02.2006 US 773068 P; 03.04.2006 US 788898 P
(43) Date of publication of application: 29.10.2008
(73) Proprietor: Oncolytics Biotech Inc., Calgary, AB T2N 1X7 (CA)
(72) Inventor: COFFEY, Matthew, C., Calgary, Alberta T2N 3L4 (CA); THOMPSON, Bradley, G., Calgary, Alberta T2N 0Z5 (CA)
(74) Representative: Nash, David Allan
(86) International application number: PCT/CA2007/000187
(87) International publication number: WO 2007/093036

(56) References cited:
- WO-A1-03/094939
- WO-A2-00/29033
- FREYTAG S ET AL: "83 Clinical trials combining oncolytic viral therapy and suicide gene therapy with conformalradiotherapy", RADIOTHERAPY AND ONCOLOGY, ELSEVIER, IRELAND, vol. 78, 1 March 2006 (2006-03-01), pages S29-S30, XP027922060, ISSN: 0167-8140, DOI: 10.1016/S0167-8140(06)80577-3 [retrieved on 2006-03-01]
- VIDAL L. ET AL.: 'A phase I study of the intratumoral administration of a wild-type reovirus in combination with radiation in cancer patients' AMERICAN ASSOCIATION FOR CANCER RESEARCH, [Online] 2005, XP008129010 Retrieved from the Internet: <URL:http://www.media.integratir.com/T.ONC/ ppt/Presentationaacr1%20slides.pdf>
- 'Oncolytic Biotech. Inc. Announces Approval for U.K. Clinical Trial Investigating REOLYSIN in Combination with Radiation Therapy' ONCOLYTICS BIOTECH. INC. PRESS RELEASE, [Online] 18 February 2005, XP008129011 Retrieved from the Internet: <URL:http://www.integratir.com/newsrelease. asp?news=2130833189&ticker=T.ONC&1ang=EN>

## Description

### TECHNICAL FIELD

The present invention relates to local immune suppression in a subject that also receives oncolytic viral therapy to enhance the efficacy of the therapy.

### BACKGROUND

Cancer is diagnosed in more than 1 million people every year in the U.S. alone. In spite of numerous advances in medical research, cancer remains the second leading cause of death in the United States. In industrialized nations, roughly one in five persons will die of cancer. In the search for novel strategies, oncolytic virus therapy has recently emerged as a viable approach to specifically kill tumor cells. Unlike conventional gene therapy, it uses replication competent viruses that are able to spread through tumor tissue by virtue of viral replication and concomitant cell lysis, providing an alternative treatment for cancer. Viruses have now been engineered to selectively replicate and kill cancer cells.

Oncolytic viruses may utilize multiple mechanisms of action to kill cancer cells- cell lysis, cell apoptosis, anti-angiogenesis and cell necrosis. The virus infects the tumor cell and then begins to replicate. The virus continues to replicate until the host cell's membrane lyses (bursts) as the tumor cell can no longer contain the virus. The tumor cell is destroyed and the newly created viruses are spread to neighboring cancer cells to continue the cycle. Oncolytic viruses are intended to replicate only in cancer cells and to pass through normal tissue without causing harm. Hence, once all the tumor cells are eradicated, the oncolytic virus no longer has the ability to replicate and the immune system clears it from the body.

Over the past few years, new insights into the molecular mechanisms of viral cytotoxicity have provided the scientific rationale to design more effective oncolytic viruses. Recent advances in molecular biology have allowed the design of several genetically modified viruses, such as adenovirus and herpes simplex virus that specifically replicate in, and kill, tumor cells. On the other hand, viruses with intrinsic oncolytic capacity are also being evaluated for therapeutic purposes. Although the efficacy of oncolytic virus therapy in general has been demonstrated in preclinical studies, the therapeutic efficacy in clinical trials is still not optimal. Therefore, strategies are evaluated that could further enhance the therapeutic potential of conditionally replicating viruses.

WO 03/094939 relates to methods of treating or ameliorating a tumor with a combination of oncolytic viruses and radiotherapy.

### SUMMARY

Provided herein is an oncolytic virus for use in a method for treating or ameliorating a solid tumor in a subject, which method comprises:
(a) injecting an effective amount of an oncolytic virus into or near the solid tumor, wherein the oncolytic virus is capable of selectively killing cells of the solid tumor, wherein the oncolytic virus is a reovirus; and
(b) suppressing local immune responses at or near the solid tumor by irradiating the solid tumor with an effective amount of an irradiating agent, wherein the amount of the irradiating agent is from 1 to 25 Gy.

In certain embodiments of the present invention, the irradiating agent is selected from the group consisting of electrons, X-ray, and gamma ray.

In certain embodiments of the present invention, the amount of the irradiating agent is 5 Gy.

In certain embodiments of the present invention, the irradiating agent is administered in one dose or fractionated over time.

In certain embodiments of the present invention, the reovirus is a mammalian reovirus or a human reovirus. In certain embodiments, the human reovirus is selected from the group consisting of serotype 1 reovirus, serotype 2 reovirus and serotype 3 reovirus. In certain embodiments, the human reovirus is serotype 3 reovirus.

In certain embodiments of the present invention, the subject is selected from the group consisting of dogs, cats, rodents, sheep, goats, cattle, horses, pigs, human and non-human primates.

In certain embodiments of the present invention, step (a) is performed prior to step (b).

### DESCRIPTION OF DRAWINGS

Figure 1 shows that as little as 5 grays (Gy) of radiation enhances reovirus cytotoxicity.
Figure 2 shows that administration of radiation and reovirus reduces tumor volume *in vivo* in immunodeficient and immunocompetent animal models.
Figure 3 shows that radiation prevents induction of neutralizing anti-virus antibodies.

### DETAILED DESCRIPTION

The terms used in this application are defined as follows unless otherwise indicated. Note the headings used herein are for organizational purposes only and are not meant to limit the description provided herein or the claims attached hereto.

### Definitions

A "neoplastic cell," "tumor cell," or "cell with a proliferative disorder" refers to a cell which proliferates at an abnormally high rate. A new growth comprising neoplastic cells is a neoplasm, also known as a "tumor." A tumor is an abnormal tissue growth, generally forming a distinct mass, that grows by cellular proliferation more rapidly than normal tissue growth. A tumor may show a partial or total lack of structural organization and functional coordination with normal tissue. As used herein, a tumor is intended to encompass hematopoietic tumors as well as solid tumors.

A tumor may be benign (benign tumor) or malignant (malignant tumor or cancer). Malignant tumors can be broadly classified into three major types. Malignant tumors arising from epithelial structures are called carcinomas; malignant tumors that originate from connective tissues such as muscle, cartilage, fat, or bone are called sarcomas; and malignant tumors affecting hematopoietic structures (structures pertaining to the formation of blood cells) including components of the immune system are called leukemias and lymphomas. Other tumors include, but are not limited to, neurofibromatosis.

A "lesion" is an injury, wound, or an area that is structurally abnormal. In the context of a subject bearing tumor, a lesion is a tumor mass unless otherwise described.

"Ras-activated neoplastic cells" or "ras-mediated neoplastic cells" refer to cells which proliferate at an abnormally high rate due to, at least in part, activation of the ras pathway. The ras pathway may be activated by way of ras gene mutation, elevated level of ras gene expression, elevated stability of the ras gene message, or any mutation or other mechanism which leads to the activation of ras or a factor or factors downstream or upstream from ras in the ras pathway, thereby increasing the ras pathway activity. For example, activation of an EGF receptor, PDGF receptor or SOS results in activation of the ras pathway. Ras-mediated neoplastic cells include, but are not limited to, ras-mediated cancer cells, which are cells proliferating in a malignant manner due to activation of the ras pathway.

"Infection by virus" refers to the entry and replication of virus in a cell. Similarly, "infection of a tumor by virus" refers to the entry and replication of virus in the cells of the tumor.

"Reovirus" refers to any virus classified in the reovirus genus, whether naturally occurring, modified, or recombinant. Reoviruses are viruses with a double-stranded, segmented RNA genome. The virions measure 60-80 nm in diameter and possess two concentric capsid shells, each of which is icosahedral. The genome consists of double-stranded RNA in 10-12 discrete segments with a total genome size of 16-27 kbp. The individual RNA segments vary in size. Three distinct but related types of reoviruses have been recovered from many species. All three types share a common complement-fixing antigen.

The human reovirus consists of three serotypes: type 1 (strain Lang or T1L), type 2 (strain Jones, T2J), and type 3 (strain Dearing or strain Abney, T3D). The three serotypes are easily identifiable on the basis of neutralization and hemagglutinin-inhibition assays (see, for example, Fields, B. N. et al., 1996).

The reovirus may be naturally occurring or modified. The reovirus is "naturally-occurring" when it can be isolated from a source in nature and has not been intentionally modified by humans in the laboratory. For example, the reovirus can be from a "field source," that is, from a human who has been infected with the reovirus. The reovirus may also be selected or mutagenized for enhanced oncolytic activity.

The reovirus may be modified but still capable of lytically infecting,a mammalian cell having an active ras pathway. The reovirus may be chemically or biochemically pretreated (e.g., by treatment with a protease, such as chymotrypsin or trypsin) prior to administration to the proliferating cells. Pretreatment with a protease removes the outer coat or capsid of the virus and may increase the infectivity of the virus. The reovirus may be coated in a liposome or micelle (Chandran and Nibert, 1998). For example, the virion may be treated with chymotrypsin in the presence of micelle-forming concentrations of alkyl sulfate detergents to generate a new infectious subvirion particle (ISVP).

The reovirus may be a recombinant reovirus resulting from the recombination/reassortment of genomic segments from two or more genetically distinct reoviruses. Recombination/reassortment of reovirus genomic segments may occur in nature following infection of a host organism with at least two genetically distinct reoviruses. Recombinant virions can also be generated in cell culture, for example, by co-infection of permissive host cells with genetically distinct reoviruses (Nibert et al. 1995).

Accordingly, the invention contemplates the use of a recombinant reovirus resulting from reassortment of genome segments from two or more genetically distinct reoviruses, including but not limited to, human reovirus, such as type 1 (e.g., strain Lang), type 2 (e.g., strain Jones), and type 3 (e.g., strain Dearing or strain Abney); non-human mammalian reoviruses; or avian reovirus. The invention further contemplates the use of recombinant reoviruses resulting from reassortment of genome segments from two or more genetically distinct reoviruses wherein at least one parental virus is genetically engineered, comprises one or more chemically synthesized genomic segment, has been treated with chemical or physical mutagens, or is itself the result of a recombination event. The invention further contemplates the use of the recombinant reovirus that has undergone recombination in the presence of chemical mutagens, including but not limited to, dimethyl sulfate and ethidium bromide, or physical mutagens, including but not limited to, ultraviolet light and other forms of radiation.

The invention further contemplates the use of recombinant reoviruses that comprise deletions or duplications in one or more genome segments, that comprise additional genetic information as a result of recombination with a host cell genome, or that comprise synthetic genes.

The reovirus may be modified by incorporation of mutated coat proteins, such as for example, into the virion outer capsid. The proteins may be mutated by replacement, insertion, or deletion. Replacement includes the insertion of different amino acids in place of the native amino acids. Insertions include the insertion of additional amino acid residues into the protein at one or more locations. Deletions include the deletion of one or more amino acid residues in the protein. Such mutations may be generated by methods known in the art. For example, oligonucleotide-site-directed mutagenesis of the gene encoding for one of the coat proteins could result in the generation of the desired mutant coat protein. Expression of the mutated protein in reovirus-infected-mammalian cells *in vitro*, such as COS 1 cells, will result in the incorporation of the mutated protein into the reovirus virion particle (Turner and Duncan, 1992; Duncan et al., 1991; Mah et al., 1990).

The reovirus is preferably a reovirus modified to reduce or eliminate an immune reaction to the reovirus. Such a modified reovirus is termed "immunoprotected reovirus." Such modifications could include packaging of the reovirus in a liposome, a micelle, or other vehicle to mask the reovirus from the immune system. Alternatively, the outer capsid of the reovirus virion particle may be removed since the proteins present in the outer capsid are the major determinant of the host humoral and cellular responses.

"Sensitizing" a neoplastic cell to radiation, as used herein, refers to the act of enhancing the sensitivity of a neoplastic cell to radiation.

"Sensitivity" of a neoplastic cell to radiation is the susceptibility of the neoplastic cell to the inhibitory effect of radiation. For example, sensitivity of a neoplastic cell to radiation may be indicated by reduction in the growth rate of the cell in response to radiation. The sensitivity may also be demonstrated by a reduction of the symptoms caused by the neoplastic cells.

A neoplastic cell that is "resistant" to radiation is a neoplastic cell not killed or growth inhibited by radiation. To determine if a neoplastic cell is growth inhibited, the growth rate of the cell in the presence or absence of radiation can be determined by established methods in the art, such as cell counts. The neoplastic cell is not growth inhibited by radiation if the growth rate is not significantly different with or without radiation.

A tumor that is "resistant" to radiation is a tumor of which the rate of size increase or weight increase does not change in the presence of radiation. Alternatively, if the subject bearing the tumor displays similar symptoms or indicators of the tumor whether the subject receives radiation or not, the tumor is resistant to radiation. For example, white cell count is commonly used as an indicator of leukemia. If the white cell count of a leukemia patient does not significantly change after receiving radiation, the leukemia of this patient is resistant to radiation.

"Administration" of a virus to a subject refers to the act of administering the virus to a subject in a manner so that it contacts the target neoplastic cells. The route by which the virus is administered, as well as the formulation, carrier or vehicle, will depend on the location as well as the type of the target cells. A wide variety of administration routes can be employed and is discussed below in further detail.

An "oncolytic virus" is a virus that preferentially replicates in, and kills, neoplastic cells. An oncolytic virus may be a naturally-occurring virus or an engineered virus. Oncolytic viruses also encompass immunoprotected and reassortant viruses as described in detail for reovirus. The virus is "naturally-occurring" when it can be isolated from a source in nature and has not been intentionally modified by humans in the laboratory. For example, the virus can be from a "field source," that is, from an infected animal. The virus is "engineered" when it has been modified by human intervention.

A "metastatic tumor" is a tumor that has metastasized from a tumor located at another place in the same animal.

An "effective amount" is an amount of an irradiating agent or reovirus which is sufficient to result in the intended effect. For an irradiating agent used to treat or ameliorate a tumor, an effective amount is an amount of the irradiating agent sufficient to alleviate or eliminate the symptoms of the tumor or to slow down the progress of the tumor.

As used herein the terms "treatment", "treat," "treating" or "ameliorating" refers to a method of reducing the effects of a disease or condition or symptom of the disease or condition. Thus in the disclosed method treatment can refer to a 10%, 20%, 30%, 40%, 50%, 60%, 70%, 80%, 90% or 100% reduction or amelioration in the severity of an established disease or condition or symptom of the disease or condition. For example, the method for treating cancer is considered to be a treatment if there is a 10% reduction in one or more symptoms of the disease in a subject as compared to control. Thus the reduction can be a 10, 20, 30, 40, 50, 60, 70, 80, 90, 100% or any percent reduction in between 10 and 100 as compared to native or control levels. It is understood that treatment does not necessarily refer to a cure or complete ablation of the disease, condition or symptoms of the disease or condition.

As used herein, the term "subject" can be a vertebrate, more specifically a mammal (e.g., a human, horse, pig, rabbit, dog, sheep, goat, non-human primate, cow, cat, guinea pig or rodent), a fish, a bird or a reptile or an amphibian. The term does not denote a particular age or sex. Thus, adult and newborn subjects, as well as fetuses, whether male or female, are intended to be covered. As used herein, patient or subject may be used interchangeably and can refer to a subject afflicted with a disease or disorder. The term patient or subject includes human and veterinary subjects.

Killing of tumor cells by replicating oncolytic viruses should provide an abundant source of tumor antigens for cross priming of host immune cells. However, anti-viral immune responses are also induced. Therefore, it is unclear whether the local killing of tumors by oncolytic viruses will be of immunotherapeutic benefit, or hindrance, to the generation of effective anti-tumor immunity.

Experiments were performed to investigate where the equilibrium between viral replication, tumor cell killing and immune reactivity (to both tumor and viral antigens) lies when tumors are undergoing effective oncolytic virotherapy. Using a model in which direct intratumoral injection of established B16 melanomas with 4 injections of reovirus (5x10⁸ pfu/injection) cures about 50% of C57B1/6 immunocompetent mice (n=8-10), extensive anti-viral immune responses were generated. However, using ELISPOT, intracellular cytokines and *in vivo* CFSE T-cell proliferation assays, it was observed that intratumoral virotherapy can indeed prime naive T cells against a defined tumor antigen. Moreover, animals cured by virotherapy also developed long term immunological memory against the tumor. These data suggest that additional approaches designed to increase the frequency of activated T cells against the tumor would increase the efficiency of oncolytic virotherapy. Therefore, oncolytic virotherapy was combined with either depletion of CD4+CD25+ Regulatory T cells (Treg) or with adoptive transfer of activated T cells specific for a tumor antigen. The results show decreased therapy efficacy in the absence of Treg and a more rapid clearance of virus replication in the treated tumor. In contrast, ongoing local virotherapy increases the attraction of activated T cells to the tumor site by over 4 fold relative to control tumors, translating into significant improvement in therapy over either virotherapy or adoptive T cell transfer therapy alone.

Thus, immune interventions which prolong local viral replication, and/or enhance frequencies of tumor specific T cells, should have significant therapeutic impact both against the local, injected tumor and against systemic metatstatic disease not accessible to direct viral injection.

Accordingly, the present invention provides, an oncolytic virus for use in a method for treating or ameliorating a solid tumor in a subject, comprising injecting an effective amount of an oncolytic virus into or near the solid tumor, wherein the oncolytic virus is capable of selectively killing cells of the solid tumor; and suppressing local immune responses at or near the site of the solid tumor. The steps of administering the oncolytic virus and suppressing local immune responses can be repeated as desired in order to ameliorate or treat the solid tumor in the subject. This method improves oncolytic virus activity within a solid tumor by antagonizing anti- viral immune responses. The ability of an oncolytic virus to mediate tumor destruction depends on the ability of the virus to replicate throughout the tumor mass. Anti-viral immunity in the tumor mass abrogates the effectiveness of oncolytic virotherapy by inhibiting the spread of the virus within the tumor. The balance between 1) viral spread and cell lysis and 2) anti- viral immune responses can be shifted to favor viral replication and spread by suppressing immune responses in the tumor. The anti-viral immune responses can be suppressed by, for example, by irradiation of the solid tumor or administration of immunosuppressive agents at or near the site of the tumor. In the method that the oncolytic virus of the invention is for use in, local anti-viral immune responses are suppressed by radiation. The immunosuppressive agent can be administered under conditions that substantially retain the immunosuppressive agent in the solid tumor. The immunosuppressive agent can be administered in multiple doses. The immunosuppressive agent can be an anti-antivirus antibody. Preferably, the immune suppression is sufficient to reduce the amount of leukocyte infiltration in the solid tumor without compromising the immune system of the mammal. This can be achieved, for example, by using proper dosage of radiation or immunosuppressive agents, and/or constraining immunosuppressive agents to the solid tumor.

Radiotherapy, also called radiation therapy, is the treatment of cancer and other diseases with radiation, typically ionizing radiation. Radiotherapy may be used to treat localized solid tumors, such as cancers of the skin, tongue, larynx, brain, breast, or uterine cervix. It can also be used to treat leukemia and lymphoma.

The amount of radiation used in radiation therapy is measured in grays (Gy), and varies depending on the type and stage of cancer being treated. For curative (radical) cases, the typical dose for a solid epithelial tumor ranges from 50 to 70 Gy, while lymphoma tumors are treated with 20 to 40 Gy. Preventative (adjuvant) doses are typically around 50 to 60 Gy in 2 Gy fractions. Usually, the total dose of radiation is fractionated over time. Typical fractionation schedules include, but are not limited to, 1.5 to 2 Gy per day.

As described herein, low doses of radiation were sufficient to improve oncolytic virus activity within a solid tumor. Specifically, as little as 5 Gy of radiation was sufficient to improve oncolytic virus activity within a solid tumor. Suitable total doses of radiation for use in the provided methods include, but are not limited to, 1, 5, 10, 15, 20 and 25 Gy of radiation or any dose of radiation between 1 and 25 Gy. The dose of radiation can be administered locally at or near the site of the tumor in one dose or can be fractionated over time.

One type of radiation therapy commonly used involves photons (electromagnetic energy). X-rays were the first form of photon radiation to be used to treat cancer. Depending on the amount of energy they possess, the rays can be used to destroy cancer cells on the surface of or deeper in the body. Linear accelerators and betatrons are machines that produce X-rays of increasingly greater energy. The use of machines to focus radiation (such as X-rays) on a cancer site is called "external beam radiotherapy."

Gamma rays are another form of photons used in radiotherapy. Gamma rays are produced spontaneously as certain elements (such as radium, uranium, and cobalt 60) release radiation as they decay. Each element decays at a specific rate and gives off energy in the form of gamma rays and other particles. X-rays and gamma rays have the same effect on cancer cells.

Another technique for delivering radiation to cancer cells is to place radioactive implants directly in a tumor or body cavity. This is called "internal radiotherapy," and brachytherapy, interstitial irradiation, and intracavitary irradiation are types of internal radiotherapy. In this treatment, the radiation dose is concentrated in a small area. Internal radiotherapy is frequently used for cancers of the tongue, uterus, and cervix.

Several new approaches to radiation therapy are being evaluated to determine their effectiveness in treating cancer. One such technique is intraoperative irradiation, in which a large dose of external radiation is directed at the tumor and surrounding tissue during surgery. Another investigational approach is particle beam radiation therapy. This type of therapy differs from photon radiotherapy in that it involves the use of fast-moving subatomic particles to treat localized cancers. A very sophisticated machine is needed to produce and accelerate the particles required for this procedure. Some particles (neutrons, pions, and heavy ions) deposit more energy along the path they take through tissue than do X-rays or gamma rays, thus causing damage to the cells they hit. This type of radiation is often referred to as high linear energy transfer (high LET) radiation. Another recent radiotherapy research has focused on the use of radiolabeled antibodies to deliver doses of radiation directly to the cancer site (radioimmunotherapy).

Methods of increasing the effectiveness of radiation therapy have been actively searched. Two, types of investigational drugs are being studied for their effects on cells undergoing radiation. Radiosensitizers make the tumor cells more likely to be damaged, and radioprotectors protect normal tissues from the effects of radiation. Hyperthermia, the use of heat, is also being studied for its effectiveness in sensitizing tissue to radiation. It is contemplated that any radiosensitizer or radioprotector known in the art can be used in conjunction with the present invention in a manner that is consistent with the present invention.

The irradiating agent useful in the method the oncolytic virus of the invention is for use in can be any irradiating agent known in the art, including but not limited to X-rays, gamma rays (e.g., gamma rays produced by radium, uranium, or cobalt 60), and particle beams (e.g., electrons, neutrons, pions, and heavy ions). The irradiation may be in the form of external radiotherapy or internal radiotherapy (including brachytherapy, interstitial irradiation, and intracavitary irradiation). The irradiating agents may be linked to an antibody, as in radioimmunotherapy, or employed during a surgery, as in intraoperative radiotherapy.

The invention involves at least a reovirus as the oncolytic virus. Oncolytic viruses include, but are not limited to, oncolytic viruses that are a member in the family of myoviridae, siphoviridae, podpviridae, teciviridae, corticoviridae, plasmaviridae, lipothrixviridae, fuselloviridae, poxyiridae, iridoviridae, phycodnaviridae, baculoviridae, herpesviridae, adnoviridae, papovaviridae, polydnaviridae, inoviridae, microviridae, geminiviridae, circoviridae, parvoviridae, hepadnaviridae, retroviridae, cyctoviridae, reoviridae, birnaviridae, paramyxoviridae, rhabdoviridae, filoviridae, orthomyxoviridae, bunyaviridae, arenaviridae, leviviridae, picornaviridae; sequiviridae, comoviridae, potyviridae, caliciviridae, astroviridae, nodaviridae, tetraviridae, tombusviridae, coronaviridae, glaviviridae, togaviridae, and barnaviridae. Immunoprotected or reassortant viruses of other oncolytic viruses are also included. Furthermore, a combination of at least two oncolytic viruses can also be employed to practice the method the oncolytic virus of the invention is for use in. A few oncolytic viruses are discussed below, and a person of ordinary skill in the art can practice the method using additional oncolytic viruses as well according to the disclosure herein and knowledge available in the art.

Normally, when a virus enters a cell, double-stranded RNA Kinase (PKR) is activated and blocks protein synthesis, and the virus cannot replicate in this cell. Some viruses have developed a system to inhibit PKR and facilitate viral protein synthesis as well as viral replication. For example, adenovirus makes a large amount of a small RNA, VA1 RNA. VA1 RNA has extensive secondary structures and binds to PKR in competition with the double-stranded RNA (dsRNA) which normally activates PKR. Since it requires a minimum length of dsRNA to activate PKR, VA1 RNA does not activate PKR. Instead, it sequesters PKR by virtue of its large amount. Consequently, protein synthesis is not blocked, and adenovirus can replicate in the cell.

Ras-activated neoplastic cells are not subject to protein synthesis inhibition by PKR because ras inactivates PKR. These cells are therefore susceptible to viral infection even if the virus does not have a PKR-inhibitory system. Accordingly, if the PKR inhibitors in adenovirus, vaccinia virus, herpex simplex virus, or parapoxvirus orf virus are mutated so as not to block PKR function anymore, the resulting viruses do not infect normal cells due to protein synthesis inhibition by PKR, but they replicate in ras- activated neoplastic cells which lack PKR activities.

Accordingly, a virus that is modified or mutated such that it does not inhibit PKR function selectively replicates in ras-activated neoplastic cells while normal cells are resistant. Preferably, the virus is an adenovirus mutated in the VA1 region, a vaccinia virus mutated in the K3L and/or E3L region, a parapoxvirus orf virus mutated in the 0V20.0L gene, or an influenza virus mutated in the NS-1 gene. The virus is preferably not a herpes virus mutated in the γ134.5 gene.

The viruses can be modified or mutated according to the known structure-function relationship of the viral PKR inhibitors. For example, since the amino terminal region of E3 protein interacts with the carboxy-terminal region domain of PKR, deletion or point mutation of this domain prevents anti-PKR function (Chang et al., 1992, 1993, 1995; Sharp et al., 1998; Romano et al., 1998). The K3L gene of vaccinia virus encodes pK3, a pseudosubstrate of PKR. There is a loss-of-function mutation within K3L. Truncations or point mutations within the C-terminal portion of K3L protein that is homologous to residues 79 to 83 in eIF-2 abolish PKR inhibitory activity (Kawagishi-Kobayashi et al., 1997).

Another example is the Delta24 virus which is a mutant adenovirus carrying a 24 base pair deletion in the E1A region (Fueyo et al., 2000). This region is responsible for binding to the cellular tumor suppressor Rb and inhibiting Rb function, thereby allowing the cellular proliferative machinery, and hence virus replication, to proceed in an uncontrolled fashion. Delta24 has a deletion in the Rb binding region and does not bind to Rb. Therefore, replication of the mutant virus is inhibited by Rb in a normal cell. However, if Rb is inactivated and the cell becomes neoplastic, Delta24 is no longer inhibited. Instead, the mutant virus replicates efficiently and lyses the Rb-deficient cell.

In addition, vesicular stomatitis virus (VSV) selectively kills neoplastic cells (and interferon can be optionally added). A herpes simplex virus 1 (HSV-1) mutant which is defective in ribonucleotide reductase expression, hrR3, was shown to replicate in colon carcinoma cells but not normal liver cells (Yoon et al., 2000). Newcastle disease virus (NDV) replicates preferentially in malignant cells, and the most commonly used strain is 73-T (Reichard et al., 1992; Zorn et al, 1994; Bar-Eli et al, 1996). Vaccinia virus propagated in several malignant tumor cell lines. Encephalitis virus was shown to have an oncolytic effect in a mouse sarcoma tumor, but attenuation may be required to reduce its infectivity in normal cells. Tumor regression has been described in tumor patients infected with herpes zoster, hepatitis virus, influenza, varicella, and measles virus (for a review, see Nemunaitis, 1999).

Sindbis virus (SIN) can be used in the methods described herein. Sindbis virus is a member of the alphavirus genus of the togaviridae family. The Sindbis virus genome is a single-stranded RNA of 11703 nucleotides, capped at the 5' terminus and poly-adenylated at the 3' terminus. The genome consists of a 49S untranslated region (UT), nonstructural proteins nsP1, nsP2, nsP3, and nsP4 followed by a promoter. The promoter is followed by a 26S UT, structural proteins C, E3, E2, 6K, and E1 and finally a 3' UT and a poly-adenylated terminus. The genomic 49S RNA is of plus sense, is infectious, and serves as mRNA in the infected cell.

Sindbis vectors systemically and specifically infect/detect and kill metastasized tumors *in vivo*, leading to significant suppression of tumor growth and enhanced survival (Hurtado et al., Rejuvenation Res. 9(1):36-44 (2006)). Sindbis virus is known to infect mammalian cells using the Mr 67,000 laminin receptor, which is elevated in tumor versus normal cells, and downregulated expression of laminin receptor with small interfering RNA significantly reduces the infectivity of Sindbis vectors. Tumor overexpression of the laminin receptor may explain the specificity and efficacy that Sindbis vectors demonstrate for tumor cells *in vivo.* Sindbis does not have to undergo genetic manipulation to target cancer cells or be injected directly into tumors. Sindbis can be injected anywhere into a subject and travel through the bloodstream to the target area (Tseng et al., Cancer Res. 64(18):6684-92 (2004). Sindbis can also be genetically engineered to carry one or more genes that suppress the immune response to the virus and/or genes that stimulate an immune response against the tumor such as, for example, antitumor cytokine genes such as interleukin-12 and interleukin-15 genes.

The oncolytic virus may be naturally occurring or modified. The virus may be chemically or biochemically pretreated (e.g., by treatment with a protease, such as chymotrypsin or trypsin) prior to administration to the neoplastic cells. Pretreatment with a protease removes the outer coat or capsid of the virus and may increase the infectivity of the virus. The virus may be coated in a liposome or micelle (Chandron and Nibert, 1998) to reduce or prevent an immune response from a mammal which has developed immunity to the virus. For example, the virion may be treated with chymotrypsin in the presence of micelle forming concentrations of alkyl sulfate detergents to generate a new infectious subvirion particle. The oncolytic virus may also be a reassortant virus or an ISVP.

It is preferable that the virus is not a vehicle for delivering a gene for the purpose of gene therapy. For example, viruses have been engineered to deliver the adenoviral E1A gene, the p53 tumor suppressor gene, prodrug-encoding genes (Chmura et al., 1999; 2001) or genes under a radiation-inducible promoter. These viruses, in fact, usually do not replicate preferentially in neoplastic cells and are hence not oncolytic viruses. It is also preferable that the virus is not an engineered adenovirus or herpes virus, or a virus that expresses a functional E1A protein.

A person of ordinary skill in the art can practice the present methods using any oncolytic virus according to the disclosure herein and knowledge available in the art. The oncolytic virus may be naturally occurring or modified. The oncolytic virus is "naturally-occurring" when it can be isolated from a source in nature and has not been intentionally modified by humans in the laboratory. For example, the oncolytic virus can be from a "field source," that is, from a human who has been infected with the oncolytic virus.

The oncolytic virus may be a recombinant oncolytic virus resulting from the recombination/reassortment of genomic segments from two or more genetically distinct oncolytic viruses. Recombination/reassortment of oncolytic virus genomic segments may occur in nature following infection of a host organism with at least two genetically distinct oncolytic virus. Recombinant virions can also be generated in cell culture, for example, by co-infection of permissive host cells with genetically distinct oncolytic viruses. The invention further contemplates the use of recombinant oncolytic virus resulting from reassortment of genome segments from two or more genetically distinct oncolytic viruses wherein at least one parental virus is genetically engineered, comprises one or more chemically synthesized genomic segment, has been treated with chemical or physical mutagens, or is itself the result of a recombination event. The invention further contemplates the use of the recombinant oncolytic virus that has undergone recombination in the presence of chemical mutagens, including but not limited to dimethyl sulfate and ethidium bromide, or physical mutagens, including but not limited to ultraviolet light and other forms of radiation.

The invention further contemplates the use of recombinant oncolytic viruses that comprise deletions or duplications in one or more genome segments, that comprise additional genetic information as a result of recombination with a host cell genome, or that comprise synthetic genes such as, for example, genes encoding agents that suppress anti-viral immune responses.

The oncolytic virus may be modified by incorporation of mutated coat proteins, such as for example, into the virion outer capsid. The proteins may be mutated by replacement, insertion or deletion. Replacement includes the insertion of different amino acids in place of the native amino acids. Insertions include the insertion of additional amino acid residues into the protein at one or more locations. Deletions include deletions of one or more amino acid residues in the protein. Such mutations may be generated by methods known in the art. For example, oligonucleotide site directed mutagenesis of the gene encoding for one of the coat proteins could result in the generation of the desired mutant coat protein. Expression of the mutated protein in oncolytic virus infected mammalian cells in vitro such as COS 1 cells can result in the incorporation of the mutated protein into the oncolytic virus virion particle.

In the provided methods, the oncolytic virus is administered in a manner so that it can ultimately contact the target tumor or tumor cells. The oncolytic virus is not administered systemically. The route by which the oncolytic virus is administered, as well as the formulation, carrier or vehicle, will depend on the location as well as the type of the target cells. A wide variety of administration routes can be employed. For example, for a solid tumor that is accessible, the oncolytic virus can be administered by injection directly to the tumor. For a hematopoietic tumor, for example, the oncolytic virus can be administered intravenously or intravascularly. For tumors that are not easily accessible within the body, such as metastases, the oncolytic virus is administered in a manner such that it can be transported systemically through the body of the mammal and thereby reach the tumor (e.g., intravenously or intramuscularly). Alternatively, the oncolytic virus can be administered directly to a single solid tumor, where it then is carried systemically through the body to metastases. The oncolytic virus can also be administered subcutaneously, intraperitoneally, intrathecally (e.g., for brain tumor), topically (e.g., for melanoma), orally (e.g., for oral or esophageal cancer), rectally (e.g., for colorectal cancer), vaginally (e.g., for cervical or vaginal cancer), nasally or by inhalation spray (e.g., for lung cancer).

Thus the herein provided viruses can be administered *in vitro* or *in vivo* in a pharmaceutically acceptable carrier. By pharmaceutically acceptable is meant a material that is not biologically or otherwise undesirable, i.e., the material may be administered to a subject, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

Thus, the invention also enables pharmaceutical compositions which contain oncolytic viruses and/or other active agents, such as, for example, a chemotherapeutic agent, as desired. In making the pharmaceutical compositions the agents are usually mixed with an excipient, diluted by an excipient or enclosed within such a carrier which can be in the form of a capsule, sachet, paper or other container. When the pharmaceutically acceptable excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions can be formulated so as to provide quick, sustained or delayed release of the active ingredient after administration to the subject by employing procedures known in the art.

For preparing solid compositions such as tablets, the active ingredient(s) is/are mixed with a pharmaceutical excipient to form a solid preformulation composition containing a homogeneous mixture of the active ingredient(s). When referring to these preformulation compositions as homogeneous, it is meant that the active ingredient is dispersed evenly throughout the composition so that the composition may be readily subdivided into equally effective unit dosage forms such as tablets, pills and capsules.

The tablets or pills may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the compositions may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described herein. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

Another formulation that can be employed in the methods provided herein employs transdermal delivery devices ("patches"). Such transdermal patches may be used to provide continuous or discontinuous infusion of the reovirus of the present invention in controlled amounts. The construction and use of transdermal patches for the delivery of pharmaceutical agents is well known in the art. See, for example, U.S. Pat. No. 5,023,252. Such patches may be constructed for continuous, pulsatile, or on demand delivery of pharmaceutical agents.

Other suitable formulations can be found in Remington's Pharmaceutical Sciences. The oncolytic virus may be packaged into convenient kits providing the necessary materials packaged into suitable containers. It is contemplated the kits may also include, agents that enhance an immune response to a tumor, agents that suppress the local immune response to the oncolytic virus, chemotherapeutic agents and/or other pharmaceutically active agents as desired.

The oncolytic virus is administered in an amount that is sufficient to treat the tumor or neoplasm (e.g., an "effective amount"). This may result in a reduction in size of the neoplasm, or in a complete elimination of the neoplasm. The reduction in size of the neoplasm, or elimination of the neoplasm, is generally caused by lysis of neoplastic cells ("oncolysis") by the oncolytic virus. Preferably the effective amount is from about 1.0 pfu/kg body weight to about 10¹⁵ pfu/kg body weight, more preferably from about 10² pfu/kg body weight to about 10¹³ pfu/kg body weight. For example, for treatment of a subject, approximately 10² to 10¹⁷ plaque forming units (PFU) of virus can be used, depending on the type, size and number of tumors present. The effective amount will be determined on an individual basis and may be based, at least in part, on consideration of the type of oncolytic virus; the chosen route of administration; the individual's size, age, gender; the severity of the patient's symptoms; the size and other characteristics of the neoplasm; and the like. The course of therapy may last from several days to several months or until diminution of the disease is achieved.

The oncolytic virus can be administered in a single dose, or multiple doses (i.e.. more than one dose). The multiple doses can be administered concurrently, or consecutively (e.g., over a period of days or weeks). The oncolytic virus can also be administered to more than one neoplasm in the same subject. The oncolytic virus can be administered prior to, along with or after the immunosuppressant. The oncolytic virus can be delivered by different routes of administration, e.g., IV, IP, etc., (sequentially or concomitantly). A course of virus and/or immunosuppressant can be administered one or more times.

The oncolytic virus is preferably formulated in a unit dosage form, each dosage containing from about 10² pfus to about 10¹⁷ pfus of the reovirus. The term "unit dosage forms" refers to physically discrete units suitable as unitary dosages for subjects, each unit containing a predetermined quantity of oncolytic virus calculated to produce the desired therapeutic effect, in association with a suitable pharmaceutical excipient.

Effective dosages of compositions depends on a variety of factors and may thus vary somewhat from subject to subject. Effective dosages and schedules for administering the compositions may be determined empirically, and making such determinations is within the skill in the art. The exact amount required will vary from subject to subject, depending on the species, age, weight and general condition of the subject, the severity of the disease being treated, the particular virus or vector used and its mode of administration. Thus, it is not possible to specify an exact amount for every composition. However, an appropriate amount can be determined by one of ordinary skill in the art using only routine experimentation given the guidance provided herein.

The dosage ranges for the administration of the compositions are those large enough to produce the desired effect in which the symptoms of the disease are affected. The dosage should not be so large as to cause adverse side effects, such as unwanted cross-reactions and anaphylactic reactions. The dosage can be adjusted by the individual physician in the event of any counter indications.

The oncolytic viruses described herein may be used for a variety of purposes. They may be used in methods for treating ras-mediated proliferative disorders in a mammal. The viruses may be used to reduce or eliminate neoplasms. They may be used in methods for treating metastases.

It is contemplated that the provided methods may be combined with other tumor therapies such as chemotherapy, radiotherapy, surgery, hormone therapy and/or immunotherapy. Thus, the oncolytic virus may be administered in conjunction with surgery or removal of the neoplasm. Therefore, provided herewith are methods for the treatment of a solid neoplasm comprising surgical removal of the neoplasm and administration of an oncolytic virus at or near to the site of the neoplasm.

It is further contemplated that the oncolytic virus may be administered in conjunction with or in addition to known anticancer compounds or chemotherapeutic agents. Chemotherapeutic agents are compounds which may inhibit the growth of tumors. Such agents, include, but are not limited to 5-fluorouracil, mitomycin C, methotrexate, hydroxyurea, cyclophosphamide, dacarbazine, mitoxantrone, anthracyclins (Epirubicin and Doxurubicin), antibodies to receptors, such as herceptin, etopside, pregnasome, platinum compounds such as carboplatin and cisplatin, taxanes such as taxol and taxotere, hormone therapies such as tamoxifen and anti-estrogens, interferons, aromatase inhibitors, progestational agents and LHRH analogs.

### Examples

### Example 1. Radiation Enhances Reovirus Cytotoxicity in vitro and in vivo

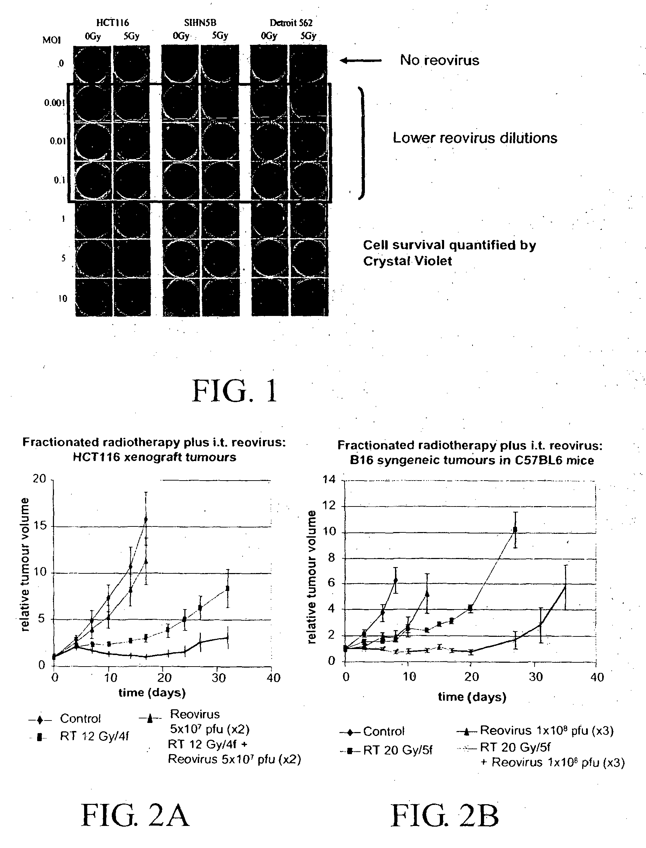

The Ras signaling pathway determines tumor cell radiosensitivity. Ras activation promotes the survival of tumor cells following radiation exposure. Ras activation is associated with increased radiation resistance *in vitro* and *in vivo*. To determine whether reovirus enhances reovirus cytotoxicity *in vitro*, cells were plated and then treated with 5 Gray or Mock treated. Four hours later serotype 3 reovirus (Dealing strain) was administered to cells. Cell survival was quantified by Crystal Violet. Figure 1 shows that as little as 5 Gy of radiation enhances reovirus cytotoxicity.

To determine the effects of radiation and reovirus *in vivo*, fractionated radiotherapy and reovirus (intrathecal administration) was administered to HCT116 xenograft tumours (Figure 2A) or B 16 syngeneic tumors (Figure 2B) in mice. Figure 2 shows that administration of radiation and reovirus reduces tumor volume *in vivo* in immunodeficient and immunocompetent animal models better than reovirus or radiation alone.

These results demonstrate that reovirus is not inactivated by clinically relevant doses of gamma-irradiation and that radiation enhances reovirus cytotoxicity. These results also demonstrated that the enhanced cytotoxicity observed is not dependent on the timing of radiation. Reovirus and radiation individually induce cell cycle arrest at G2/M and together cause increased levels of apoptosis.

### Example 2. Effects of Reovirus and Radiation Therapy in Humans

The study design for determining the effects of reovirus and radiation therapy in humans is shown in Table 1.

**Table 1. Study Design**

| **Phase** | **Patients x Cohort** | **Reolysin Dose (TCID50)** | **RT (Gy)** | **Virus Dose** | **Day of Virus Administration** |
|---|---|---|---|---|---|
| Ia | 3 | 1 x 10⁸ | 20 Gy in 5F | 2 | 2, 4 |
| Ia | 3 | 1 x 10⁹ | 20 Gy in 5F | 2 | 2, 4 |

Patients were included based on a measurable lesion (target lesion), palliative RT indicated, PS (ECOG) ≤ 2, adequate haematological, renal and hepatic function. Patients with lesions previously irradiated or patients on immunosuppressive therapy or with known HIV, Hep B or C infection were excluded.

The amount of reolysin depended on the target volume of the lesion. Tumor volume was calculated by measuring three orthogonal diameters (D1, D2 and D3) and applying the formula V=π/6 x D1 x D2 x D3. Tumors received injection volumes between 2 to 10 mls.

Radiation was delivered with electrons or orthovoltage X-rays based on gross tumor volume. The gross tumor volume was delineated in 3-dimensions by clinical and/or radiological examination. A circumferential margin of 1 cm was added to comprise the planning target volume.

Dose-limiting toxicity (DLT) was determined as follows: ANC < 0.5 x 10⁹ lasting for more than five days or ANC < 0.5 x 10⁹ with sepsis; platelet count < 25 x 10⁹/L; grade 2 neurotoxicity or cardiotoxicity; or any other drug-related non-haematological grade 3/4 toxicity, with the exceptions of flu-like symptoms, nausea and vomiting if appropriate prophylactic or therapeutic measures have not been administered.

The patient summary is shown in Table 2.

**Table 2. Patient Summary**

| **Tumor Type** | **Age** | **Sex** | **Target Lesion** | **Reovirus Dose (TCID50)** | **Radiation Dose** |
|---|---|---|---|---|---|
| Oesophageal | 56 | M | Supraclavicular lymph node | 1 x 10⁸ | 20 Gy |
| Melanoma | 58 | M | Nodule on the groin | 1 x 10⁸ | 20 Gy |
| Pancreas | 67 | M | Abdominal wall mass | 1 x 10⁸ | 20 Gy |
| Ovarian* | 58 | F | Nodule on the back | 1 x 10⁹ | 20 Gy |
| Unknown Primary Squamous Cell | 41 | F | Nodule on the clavicle | 1 x 10⁹ | 20 Gy |
| Squamous Cell of the Scalp | 71 | M | Left parotid | 1 x 10⁹ | 20 Gy |
| Small Cell Lung Cancer | 70 | M | Nodule on the arm | 1 x 10⁹ | 20 Gy |

| | | | | | |
|---|---|---|---|---|---|
| *Patient only received one dose of reovirus. | | | | | |

To measure reovirus excretion, samples from subjects were taken pre- and posttreatment. RNA was extracted from blood, urine, stool and sputum. RT-PCR analysis showed that no viral shedding was detected in these subjects at any dose level.

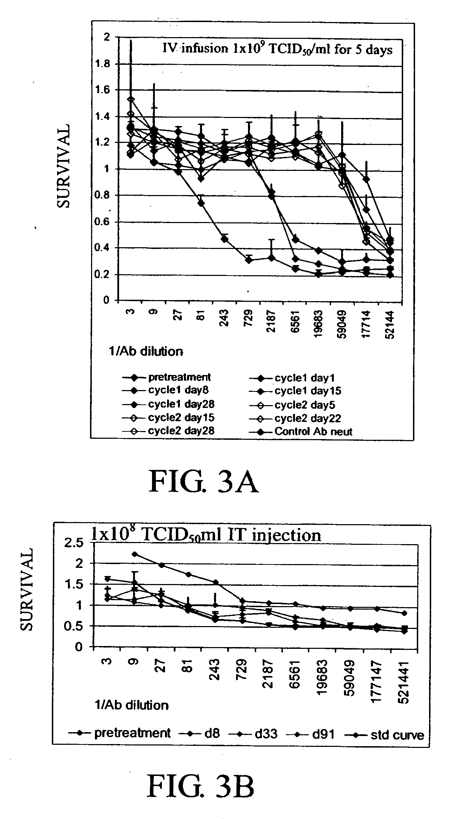

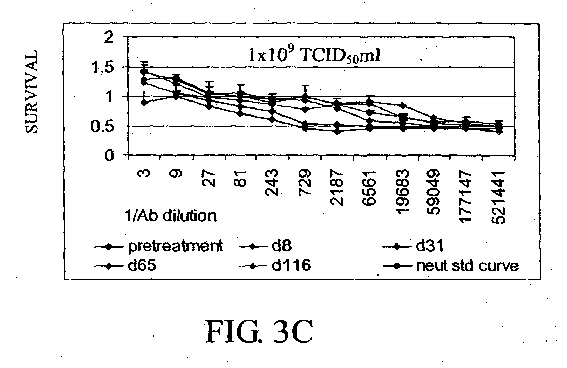

To determine whether radiation suppresses local immune responses levels of neutralzing anti-virus antibody were examined. Figure 3 shows that radiation prevents induction of neutralizing anti-virus antibodies.

Table 3 shows the anti-tumor activity of reovirus and radiation in the target lesion.

**Table 3. Anti-Tumor Activity in Target Lesion**

| **Tumor Type** | **Target Lesion** | **Response 1 Month** | **Response 2 Months** | **Response 3 Months** |
|---|---|---|---|---|
| Oesophageal | Supraclavicular lymph node | PR | PR | PR |
| Melanoma | Nodule in the groin | SD | - | - |
| Pancreas | Abdominal wall mass | PD | - | - |
| Unknown Primary Squamous Cell | Nodule on clavicle | PR | PR | PR |
| Squamous Cell Scalp | Left parotid | SD | SD | pPR |

| | | | | |
|---|---|---|---|---|
| PR-partial response; SD-stable disease; PD-progressive disease; pPR-pathologic partial response | | | | |

These results show that the combination of intratumoral reovirus and radiation is well tolerated. In addition, no DLT, viral shedding, or induction of neutralizing anti-reovirus antibodies were observed.

### References

U.S. Pat. No. 6,136,307.
U.S. Pat. No. 6,100,243.
U.S. Patent Application Publication No. 20020037576.
Bar-Eli, N., et al., "Preferential cytotoxic effect of Newcastle disease virus on lymphoma cells," J. Cancer Res. Clin. Oncol. 122: 409-415 (1996).
Chandran and Nibert, "Protease cleavage of reovirus capsid protein mu1 and mulC is blocked by alkyl sulfate detergents, yielding a new type of infectious subvirion particle," J. of Virology 72(1):467-75 (1998).
Chang et al., PNAS 89:4825-4829 (1992).
Chang, H. W. et al., Virology 194:537-547 (1993).
Chang et al., J. Virol. 69:6605-6608 (1995).
Chmura et al., "Strategies for enhancing viral-based gene therapy using ionizing radiation," Radiation oncology Investigations 7:261-269 (1999).
Chmura et al., "Prospects for viral-based strategies enhancing the anti-tumor effects of ionizing radiation," Seminars in Radiation Oncology 11(4):338-345 (2001).
Cuff et al., "Enteric reovirus infection as a probe to study immunotoxicity of the gastrointestinal tract," Toxicological Sciences 42(2):99-108 (1998).
Duncan et al., "Conformational and functional analysis of the C-terminal globular head of the reovirus cell attachment protein," Virology 182(2):810-9 (1991).
Fields, B. N. et al., Fundamental Virology, 3rd Edition, Lippincott-Raven (1996).
Fueyo, J., et al., "A Mutant Oncolytic Adenovirus Targeting the Rb Pathway Produces Anti-Glioma Effect in vivo," Oncogene 19(1):2-12 (2000).
Harlow et al., "Antibodies. A Laboratory Manual," Cold Spring Harbor Laboratory, New York, 1988.
Kawagishi-Kobayashi, M., et al., Mol. Cell. Biology 17:4146-4158 (1997).
Mah et al., "The N-terminal quarter of reovirus cell attachment protein sigma 1 possesses intrinsic virion-anchoring function," Virology 179(1):95-103 (1990).
Nemunaitis, J., Invest. New Drugs 17:375-386 (1999).
Nibert, M. L., Schiff, L. A., and Fields, B. N., "Reoviruses and their replication," pages 1557-96 in Fundamental Virology (Fields et al., 3rd Edition), Lippincott-Raven Press, 1996.
Reichard, K. W., et al., "Newcastle disease virus selectively kills human tumor cells," J. of Surgical Research 52:448-453 (1992).
Romano et al., Mol. and Cell. Bio. 18:7304-7316 (1998).
Sharp et al., Virol. 250:301-315 (1998).
Turner and Duncan, "Site directed mutagenesis of the C-terminal portion of reovirus protein sigmal : evidence for a conformation-dependent receptor binding domain," Virology 186(1):219-27 (1992).
Yoon, S. S., et al., "An oncolytic herpes simplex virus type I selectively destroys diffuse liver metastases from colon carcinoma," FASEB J. 14:301-311(2000).
Zorn, U. et al., "Induction of cytokines and cytotoxicity against tumor cells by newcastle disease virus," Cancer Biotherapy 9(3):22-235 (1994).

## Claims

1. An oncolytic virus for use in a method for treating or ameliorating a solid tumor in a subject, which method comprises:
(a) injecting an effective amount of an oncolytic virus into or near the solid tumor, wherein the oncolytic virus is capable of selectively killing cells of the solid tumor, wherein the oncolytic virus is a reovirus; and
(b) suppressing local immune responses at or near the solid tumor by irradiating the solid tumor with an effective amount of an irradiating agent, wherein the amount of the irradiating agent is from 1 to 25 Gy.

2. The oncolytic virus for use of claim 1, wherein the irradiating agent is selected from the group consisting of electrons, X-ray, and gamma ray.

3. The oncolytic virus for use of claim 1 or 2, wherein the amount of the irradiating agent is 5 Gy.

4. The oncolytic virus for use of any one of claims 1 to 3, wherein the irradiating agent is administered in one dose or fractionated over time.

5. The oncolytic virus for use of any one of claims 1 to 4, wherein the reovirus is a mammalian reovirus or a human reovirus.

6. The oncolytic virus for use of claim 5, wherein the human reovirus is selected from the group consisting of serotype 1 reovirus, serotype 2 reovirus and serotype 3 reovirus.

7. The oncolytic virus for use of claim 6, wherein the human reovirus is serotype 3 reovirus.

8. The oncolytic virus for use of any one of claims 1 to 7, wherein the subject is selected from the group consisting of dogs, cats, rodents, sheep, goats, cattle, horses, pigs, human and non-human primates.

9. The oncolytic virus for use of any one of claims 1 to 8, wherein step (a) is performed prior to step (b).

## Patentansprüche

1. Onkolytisches Virus zur Verwendung in einem Verfahren zum Behandeln oder Verbessern eines massiven Tumors in einem Subjekten, wobei das Verfahren umfasst
(a) Injektion einer wirksamen Menge des onkolytischen Virus in den oder in die Nähe des massiven Tumors, wobei das onkolytische Virus fähig ist, selektiv Zellen des massiven Tumors zu töten, wobei das onkolytische Virus ein Reovirus ist; und
(b) Unterdrückung lokaler Immunreaktionen am oder in der Nähe des massiven Tumors durch Bestrahlung des massiven Tumors mit einer wirksamen Menge eines Bestrahlungsmittels, wobei die Menge des Bestrahlungsmittels von 1 bis 25 Gy ist.

2. Onkolytisches Virus zur Verwendung gemäß Anspruch 1, wobei das Bestrahlungsmittel ausgewählt ist aus der Gruppe Elektronen, Röntgenstrahlung und Gammastrahlung.

3. Onkolytisches Virus zur Verwendung gemäß Anspruch 1 oder 2, wobei die Menge des Bestrahlungsmittels 5 Gy ist.

4. Onkolytisches Virus zur Verwendung gemäß irgendeinem der Ansprüche 1 bis 3, wobei das Bestrahlungsmittel in einer Dosis oder über die Zeit fraktioniert verabreicht wird.

5. Onkolytisches Virus zur Verwendung gemäß irgendeinem der Ansprüche 1 bis 4, wobei das Reovirus ein Säugetier-Reovirus oder ein menschliches Reovirus ist.

6. Onkolytisches Virus zur Verwendung gemäß Anspruch 5, wobei das menschliche Reovirus ausgewählt ist aus der Gruppe Serotyp 1-Reovirus, Serotyp 2-Reovirus und Serotyp 3-Reovirus.

7. Onkolytisches Virus zur Verwendung gemäß Anspruch 6, wobei das menschliche Reovirus Serotyp 3-Reovirus ist.

8. Onkolytisches Virus zur Verwendung gemäß irgendeinem der Ansprüche 1 bis 7, wobei das Subjekt ausgewählt ist aus der Gruppe Hunde, Katzen, Nagetiere, Schafe, Ziegen, Rinder, Pferde, Schweine, menschliche und nicht-menschliche Primaten.

9. Onkolytisches Virus zur Verwendung gemäß irgendeinem der Ansprüche 1 bis 8, wobei Schritt (a) vor Schritt (b) ausgeführt wird.

## Revendications

1. Un virus oncolytique destiné à une utilisation dans un procédé de traitement ou d'amélioration d'une tumeur solide chez un sujet, ledit procédé comprenant :
(a) l'injection d'une quantité efficace d'un virus oncolytique dans ou près de la tumeur solide, où le virus oncolytique est capable de tuer de manière sélective des cellules de la tumeur solide, où le virus oncolytique est un réovirus ; et
(b) la suppression de réponses immunitaires locales au niveau ou près de la tumeur solide par l'irradiation de la tumeur solide avec une quantité efficace d'un agent d'irradiation, où la quantité de l'agent d'irradiation est de 1 à 25 Gy.

2. Le virus oncolytique destiné à une utilisation selon la revendication 1, où l'agent d'irradiation est sélectionné dans le groupe se composant d'électrons, de rayons x et de rayons gamma.

3. Le virus oncolytique destiné à une utilisation selon la revendication 1 ou 2, où la quantité de l'agent d'irradiation est de 5 Gy.

4. Le virus oncolytique destiné à une utilisation selon l'une quelconque des revendications 1 à 3, où l'irradiation est administrée en une seule dose ou de manière fractionnée au fil du temps.

5. Le virus oncolytique destiné à une utilisation selon l'une quelconque des revendications 1 à 4, où le réovirus est un réovirus de mammifère ou un réovirus humain.

6. Le virus oncolytique destiné à une utilisation selon la revendication 5, où le réovirus humain est sélectionné dans le groupe se composant de réovirus de sérotype 1, réovirus de sérotype 2 et réovirus de sérotype 3.

7. Le virus oncolytique destiné à une utilisation selon la revendication 6, où le réovirus humain est un réovirus de sérotype 3.

8. Le virus oncolytique destiné à une utilisation selon l'une quelconque des revendications 1 à 7, où le sujet est sélectionné dans le groupe se composant de chiens, chats, rongeurs, moutons, chèvres, bovins, chevaux, porcs, primates humains et non humains.

9. Le virus oncolytique destiné à une utilisation selon l'une quelconque des revendications 1 à 8, où l'opération (a) est exécutée avant l'opération (b).
